# EUROPEAN PATENT SPECIFICATION

(11) **EP 4 327 745 B1**
(45) Date of publication and mention of the grant of the patent: **25.12.2024**
(21) Application number: 23191781.6
(22) Date of filing: 16.08.2023
(51) Int. Cl.: A61B 5/349, A61B 5/00

(54) **ELECTRONIC DEVICE AND METHOD FOR DIAGNOSING HEART STATE BASED ON ELECTROCARDIOGRAM**
ELEKTRONISCHE VORRICHTUNG UND VERFAHREN ZUR DIAGNOSE DES HERZZUSTANDS AUF BASIS EINES ELEKTROKARDIOGRAMMS
DISPOSITIF ÉLECTRONIQUE ET PROCÉDÉ DE DIAGNOSTIC D'ÉTAT CARDIAQUE SUR LA BASE D'ÉLECTROCARDIOGRAMME

(30) Priority: 18.08.2022 TW 111131071
(43) Date of publication of application: 28.02.2024
(73) Proprietor: Acer Incorporated, New Taipei City 221 (TW); Acer Medical Inc., New Taipei City 22181 (TW); National Health Research Institutes, Zhunan Town 350 Miaoli County (TW); Chang Gung Memorial Hospital, Keelung, Anle Dist. 20401 Keelung City (TW)
(72) Inventor: CHEN, Jun-Hong, c/o Acer Incorporated, New Taipei City 221, Taiwan ( R.O.C.) (TW); CHU, Sheng-Wei, c/o Acer Incorporated, New Taipei City 221, Taiwan ( R.O.C.) (TW); LIN, Pin-Cyuan, c/o Acer Incorporated, New Taipei City 221, Taiwan ( R.O.C.) (TW); LIN, Yi-Chun, c/o Acer Medical Inc., New Taipei City 22181, Taiwan ( R.O.C.) (TW); YEH, Chi-Hsiao, c/o Chang Gung Memorial Hospital, Keelung, Keelung City 20401 (TW); TSAI, Ting-Fen, c/o National Health Research Institutes, Miaoli County 350 (TW)
(74) Representative: Haseltine Lake Kempner LLP

(56) References cited:
- PRIM JOSHUA ET AL: "A Data Pipeline for Extraction and Processing of Electrocardiogram Recordings", 2021 COMPUTING IN CARDIOLOGY (CINC), 13 September 2021 (2021-09-13), pages 1 - 4, XP093120425, Retrieved from the Internet <URL:https://ieeexplore.ieee.org/abstract/document/9662946> [retrieved on 20240117], DOI: 10.22489/CinC.2021.228
- JENKS DAVID MATTHEW: "A Deep Learning Approach to Heartbeat Classification", MASTER THESIS, 1 December 2016 (2016-12-01), pages 1 - 98, XP093119390, Retrieved from the Internet <URL:https://scholarworks.calstate.edu/downloads/vh53wz51k>
- JAXY SIMON: "Teaching a Machine to Diagnose a Heart Disease", BACHELOR THESIS, 3 September 2020 (2020-09-03), pages 1 - 56, XP093119356, Retrieved from the Internet <URL:https://arxiv.org/pdf/2009.01076.pdf> DOI: https://doi.org/10.48550/arXiv.2009.01076

## Description

### BACKGROUND

### Technical Field

The disclosure relates to an electrocardiogram analysis method, and in particular to a method and an electronic device for diagnosing heart state based on electrocardiogram.

### Description of Related Art

Cardiovascular disease has been one of the top ten causes of death for many years. Since cardiovascular disease has no obvious symptoms, it poses a great threat to patients. The oxygen needed by the heart is mainly supplied by the three coronary arteries. When any of the coronary arteries supplying myocardial blood is narrowed or blocked, the oxygen and nutrient supply to the heart will be blocked, resulting in myocardial hypoxia, that is, coronary heart disease (CAD), which may lead to patient death in severe cases.

In order to check coronary artery related diseases, doctors often use electrocardiogram (ECG) for non-invasive examination. Currently, physicians often use 12-Lead ECG for initial judgment. When performing electrocardiogram measurement, medical staff will stick patches on the patient's limbs and chest to measure the ECG signal. Currently, each lead is actually measured for 10 seconds, but the electrocardiogram report in a PDF file format provided by the medical equipment will only display the signal diagram for 2.5 seconds for most leads. That is to say, 10 seconds raw signal value of the complete 12 lead will not be presented in the electrocardiogram report in the PDF file format. In addition, although there are many documents disclosing the salient features of CAD patients in electrocardiogram, such as ST elevation, there are still hidden features in electrocardiogram that are difficult to judge with naked eyes. Therefore, physicians often need further invasive cardiac catheterization for confirmation. However, cardiac catheterization is an invasive procedure with risks and complications. Therefore, how to propose an electrocardiogram analysis method that can effectively assist medical personnel in making disease decisions is one of the goals that people in the field are committed to. Prim Joshua ET AL ("A Data Pipeline for Extraction and Processing of Electrocardiogram Recordings", 2021 Computing in Cardiology (CinC), 2021-09-13), Jenks David Matthew ("A Deep Learning Approach to Heartbeat Classification", Master Thesis, 2016-12-01), and Jaxy Simon ("Teaching a Machine to Diagnose a Heart Disease", Bachelor Thesis, 2020-09-03) suggest various approaches for an electrocardiogram analysis.

### SUMMARY

Accordingly, the present disclosure provides a method and electronic device for diagnosing a heart state based on an electrocardiogram, which can generate a diagnostic result to assist medical personnel in decision-making according to an electrocardiogram file in a common file format.

A method of diagnosing a heart state based on an electrocardiogram according to an embodiment of the present disclosure includes the following steps: obtaining an electrocardiogram file, wherein the electrocardiogram file is in a first file format and comprises a plurality of potential traces of a plurality of leads; converting the electrocardiogram file into a second file format to obtain electrocardiogram data corresponding to the plurality of leads, wherein, each of the plurality of potential traces relative to time in the electrocardiogram file is converted into the electrocardiogram data of each of the plurality of lead; generating an integrated electrocardiogram data associated with the plurality of leads based on the electrocardiogram data of the plurality of leads through a zero-padding operation and a stacking operation; and generating a diagnostic result of a heart state according to the integrated electrocardiogram data and a deep learning model.

An electronic device according to an embodiment of the present disclosure includes a storage device and a processor. The processor is coupled to the storage device and configured to: obtaining an electrocardiogram file, wherein the electrocardiogram file is a first file format; converting the electrocardiogram file into a second file format to obtain electrocardiogram data corresponding to a plurality of leads, wherein the electrocardiogram data of each of the plurality of leads comprises a potential trace relative to time; generating an integrated electrocardiogram data associated with the plurality of leads based on the electrocardiogram data of the plurality of leads through a zero-padding operation and a stacking operation; and generating a diagnostic result of a heart state according to the integrated electrocardiogram data and a deep learning model.

Based on the above, in the embodiment of the present disclosure, the electrocardiogram file in the first file format can be converted to the second file format, so as to obtain electrocardiogram data corresponding to a plurality of leads from a file in the second file format. A zero-padding operation and a stacking operation is performed on the electrocardiogram data of the leads can generate an integrated ECG data. Therefore, the deep learning model can predict the diagnostic result of the heart state based on the integrated ECG data, and medical personnel can more accurately evaluate the patient's heart state based on the diagnostic result output by the deep learning model.

### BRIEF DESCRIPTION OF THE DRAWINGS

FIG. 1 is a schematic diagram of an electronic device according to an embodiment of the present disclosure.
FIG. 2 is a flowchart of a method for diagnosing a heart state based on an electrocardiogram according to an embodiment of the present disclosure.
FIG. 3 is a schematic diagram of an electrocardiogram file according to an embodiment of the present disclosure.
FIG.4A and FIG. 4B are schematic diagrams of a zero-padding operation and a stacking operation according to an embodiment of the present disclosure.
FIG. 5 is a flowchart of a method for diagnosing a heart state based on an electrocardiogram according to an embodiment of the present disclosure.
FIG. 6 is a schematic diagram of a deep learning model according to an embodiment of the present disclosure.
FIG. 7 is a schematic diagram of a training depth model and an application depth model according to an embodiment of the present disclosure.

### DESCRIPTION OF THE EMBODIMENTS

In order to make the content of the present disclosure more comprehensible, the following specific embodiments are taken as examples in which the present disclosure can actually be implemented. In addition, wherever possible, elements/components/steps using the same reference numerals in the drawings and embodiments represent the same or similar parts.

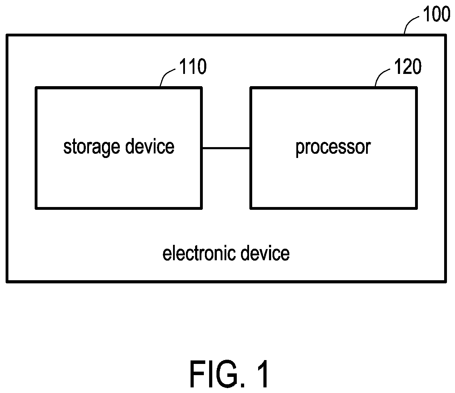

FIG. 1 shows a schematic diagram of an electronic device 100 for diagnosing a heart state based on an electrocardiogram (ECG) according to an embodiment of the present disclosure. The electronic device 100 may include a storage device 110 and a processor 120.

The storage device 110 is configured to store data such as images, data, and codes (such as operating systems, applications, and drivers) accessed by the processor 120, which can be, for example, any type of fixed or removable random access memory (RAM), read-only memory (ROM), flash memory, hard disk, solid state drive (SSD) or similar components or the above components combination.

The processor 120 is coupled to the storage device 110, such as a central processing unit (CPU), an application processor (AP), or other programmable general-purpose or special-purpose microprocessor, digital signal processor (DSP), image signal processor (ISP), graphics processing unit (GPU) or other similar devices, integrated circuits or combinations thereof. The processor 120 can access and execute the instructions, program codes and software modules recorded in the storage device 110 to implement the method based on electrocardiogram diagnose heart state in the embodiment of the present disclosure. The following examples illustrate the detailed steps of the electronic device 100 executing the method based on electrocardiogram diagnose heart state.

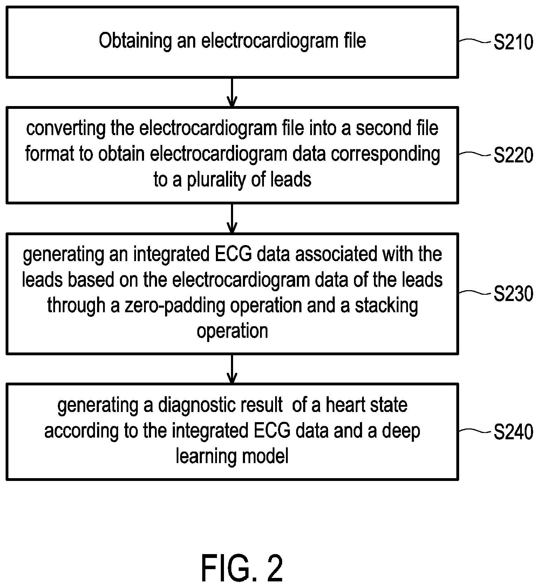

FIG. 2 is a flowchart of a method for diagnosing a heart state based on an electrocardiogram according to an embodiment of the present disclosure. Please refer to FIG. 1 and FIG. 2, the method of this embodiment is applicable to the electronic device 100 in the above-mentioned embodiment, and the details of each step in FIG. 2 will be described below with each component in the electronic device 100.

In step S210, the processor 120 obtains an electrocardiogram file. This electrocardiogram file is in a first file format and includes a plurality of potential traces of a plurality of leads. The electrocardiogram (ECG) records the electrical activity of the heart in time units by capturing the electrical potential transmission of the heart through electrodes in contact with the skin. In some embodiments, the electrocardiogram measuring device may generate an electrocardiogram file in a first file format. The processor 120 can obtain the electrocardiogram file generated by the electrocardiogram measurement equipment through wired or wireless data transmission technology. In some embodiments, the first file format may be a portable document format (PDF) file format, and these leads may include at least two of the followings: lead I, lead II, lead III, lead aVR, lead aVL, lead aVF, lead V1 , lead V2, lead V3, lead V4, lead V5, lead V6.

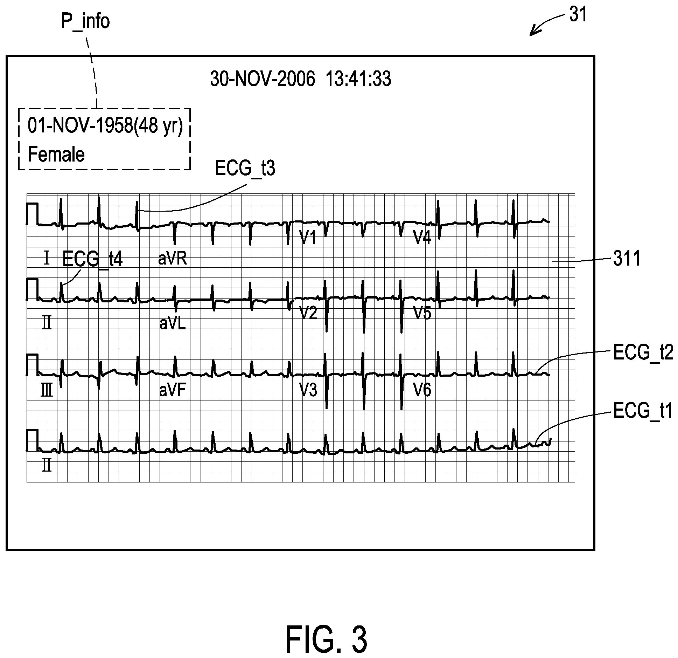

For example, FIG. 3 is a schematic diagram of an electrocardiogram file according to an embodiment of the present disclosure. Assume that the electrocardiogram file 31 is a PDF file, and patient data P_info and electrocardiogram 311 are recorded. In this embodiment, the electrocardiogram 311 may be a 12 lead (also called 12-lead) electrocardiogram, but the present disclosure is not limited thereto. The horizontal axis of the electrocardiogram 311 represents time in milliseconds (ms), and the vertical axis of the electrocardiogram 311 represents potential difference in millivolts (mV). Electrocardiogram 311 records the potential trace of a plurality of leads relative to time, and these leads may include lead I, lead II, lead III, lead aVR, lead aVL, lead aVF, lead V1, lead V2, lead V3, lead V4, lead V5 and lead V6. For example, electrocardiogram 311 includes potential trace ECG_t3 of lead I, potential trace ECG_t2 of lead V6, and potential trace ECG_t1 of lead II. However, although FIG. 3 is illustrated with 12-lead as an example, the present disclosure is not limited thereto. In some embodiments, the electrocardiogram file records potential traces of at least two leads among the 12 leads.

In step S220, the processor 120 converts the electrocardiogram file into a second file format to obtain electrocardiogram data corresponding to the plurality of leads. Wherein, each potential trace relative to time in the electrocardiogram 311 of the electrocardiogram file converts into the electrocardiogram data of each lead. Specifically, the electrocardiogram data of each lead may include a plurality of potential values corresponding to a plurality of sampling time.

In some embodiments, the second file format includes a scalable sector graphics (SVG) file format, or other vector graphics formats. The SVG file format complies with the Extensible Markup Language (XML) syntax, and uses a descriptive language in text format to describe image content, so it is a vector graphics format that has nothing to do with image resolution. Specifically, by converting the PDF file into an SVG file, the processor 120 can convert the electrocardiogram file into a second file format. It should be noted that converting the PDF file format into the SVG file format is a known technology, and will not be repeated herein. It should be noted that, in some embodiments, by converting the electrocardiogram file into a vector graphics format, the processor 120 can restore the potential trace of each lead in the electrocardiogram file to a plurality of potential values corresponding to multiple sampling time.

In detail, the file converted to graphic vector format can describe the potential trace of each lead in the electrocardiogram (electrocardiogram 311 shown in FIG. 3) in a descriptive language in text format. Therefore, after obtaining the file converted to the second file format, the processor 120 can obtain the trace description coordinate data corresponding to each lead from the file converted to the second file format according to a label (also called element label) defined by the second file format. It is known that the trace description coordinate data of each lead can be used to describe the potential trace of each lead. For example, the processor 120 can extract the trace description coordinate data used to describe the potential trace of each lead in the electrocardiogram according to the label <path> in the SVG file. In detail, since the position where the potential trace of each lead is displayed in the electrocardiogram file can be known information, the processor 120 can use the label in the SVG file<path> And the display location information of the potential trace of each lead to retrieve the trace description coordinate data of the potential trace of each lead.

Then, in some embodiments, the processor 120 can convert the trace description coordinate data of each lead into an electrocardiogram data corresponding to each lead. Specifically, the processor 120 can convert the trace description coordinate data used to describe the electrocardiogram signal waveform, i.e., the potential trace of each lead, into a plurality of potential values in units of millivolts (mV) and corresponding to a plurality of sampling time.

In step S230, the processor 120 generates an integrated ECG data associated with the plurality of leads based on the electrocardiogram data of the plurality of leads through the zero-padding operation and the stacking operation. In detail, the potential trace of most leads in the electrocardiogram file will correspond to a partial period (period of time), and the potential trace of at least one lead in the electrocardiogram file will correspond to a complete period. In some embodiments, the length of the complete period is, for example, 10 seconds, and the above-mentioned partial periods are, for example, 0 to 2.5 seconds, 2.5 to 5 seconds, 5 to 7.5 seconds, and 7.5 to 10 seconds. Since the potential trace of partial leads is corresponding to the partial period, the processor 120 can perform the zero-padding operation on the electrocardiogram data of these partial leads corresponding to the partial period, so that a data size of the compensated ECG data of each lead corresponding to the partial period is the same as a data size of the electrocardiogram data of at least one lead corresponding to the complete period.

Taking FIG. 3 as an example, the electrocardiogram file 31 output by the electrocardiogram measuring device includes 12-lead electrocardiograms. Among them, the potential trace ECG_t1 of the lead II at the bottom of the electrocardiogram 311 corresponds to the complete period "0 to 10 seconds". The potential trace ECG_t3 of lead I corresponds to the partial period "0 to 2.5 seconds". The potential trace ECG_t2 of lead V3 corresponds to the partial period "7.5 seconds to 10 seconds", so on and so forth. It should be noted that the electrocardiogram 311 also includes the potential trace ECG_t4 of the lead II corresponding to the partial period "0 to 2.5 seconds". Accordingly, after obtaining the electrocardiogram data of each lead, the processor 120 will perform zero-padding operation to the electrocardiogram data of lead I, lead II, lead III, lead aVR, lead aVL, lead aVF, lead V1, lead V2, lead V3, lead V4, lead V5, and lead 6. However, FIG. 3 is merely an exemplary illustration, and is not intended to limit the present disclosure.

In some embodiments, the plurality of leads include a first lead and a second lead. The processor 120 may perform the zero-padding operation in at least one second time span other than the first time span based on the first time span corresponding to the electrocardiogram data of the first lead to generate the compensated ECG data of the first lead. The third time span corresponding to the electrocardiogram data of the second lead includes the first time span and the at least one second time span. In detail, the processor 120 determines the at least one second time span for performing the zero-padding operation according to the first time span (partial period) corresponding to the electrocardiogram data of the first lead and the third time span (complete period) corresponding to the electrocardiogram data of the second lead.

In addition, it is noted that, in some embodiments, the plurality of leads further include a third lead. Based on the fourth time span corresponding to the electrocardiogram data of the third lead, the processor 120 may perform the zero-padding operation in at least one fifth time span other than the fourth time span to generate the compensated ECG data of the third lead. It should be noted that the at least one second time span associated with the first lead does not partially overlap with the at least one fifth time span associated with the third lead. In detail, based on the zero-padding operation similar to that of the first lead, the processor 120 can determine the at least one fifth time span for performing the zero-padding operation according to the fourth time span (partial period) corresponding to the electrocardiogram data of the third lead and the third time span (complete period) corresponding to the electrocardiogram data of the second lead. The third time span corresponding to the electrocardiogram data of the second lead includes the first time span and the at least one fifth time span. Moreover, if the first lead and the third lead correspond to different partial periods, the second period and the fifth period for performing the zero-padding operation do not partially overlap with each other.

In some embodiments, the processor 120 can stack the compensated ECG data of the first lead and the electrocardiogram data of the second lead to generate an integrated ECG data associated with the plurality of leads. Specifically, after the compensating operation is performed, the data size of the compensated ECG data corresponding to each lead of the partial period may be the same as the data size of the electrocardiogram data corresponding to at least one lead of the complete period. Specifically, both the compensated ECG data of the first lead and the electrocardiogram data of the second lead can both be a 1*S data array, where S is the sampling amount in the complete period. For example, if the duration of the complete period is 10 seconds and the sampling rate is 500 samples per second, then S=5000. Accordingly, the processor 120 can stack the compensated ECG data of the first lead and the electrocardiogram data of the second lead to generate 2*S integrated ECG data. In other words, the integrated ECG data may be a stacked data array generated by the processor 120 stacking a plurality of data arrays with the same data size.

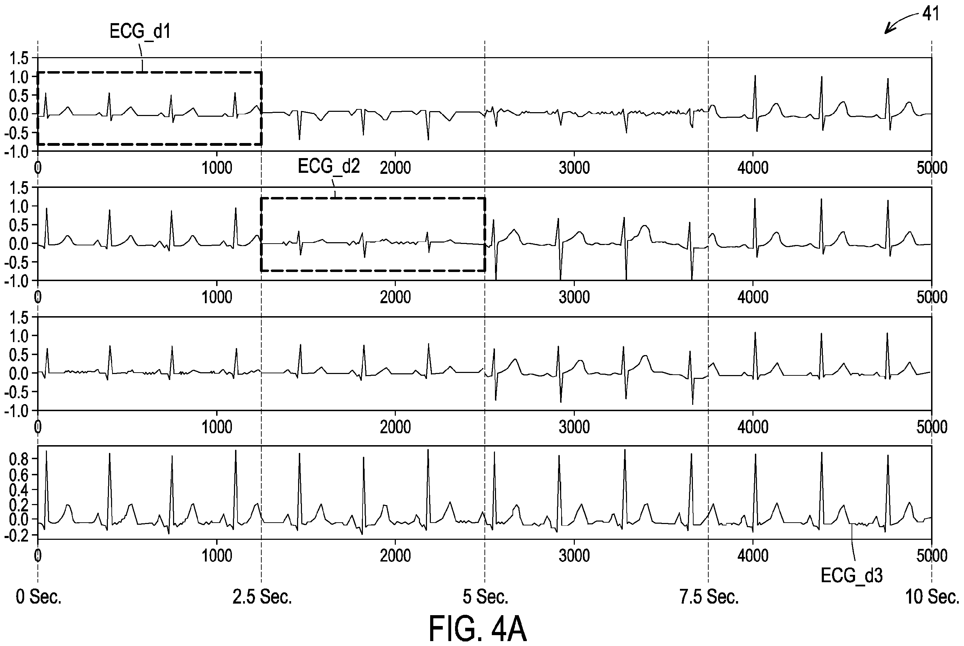

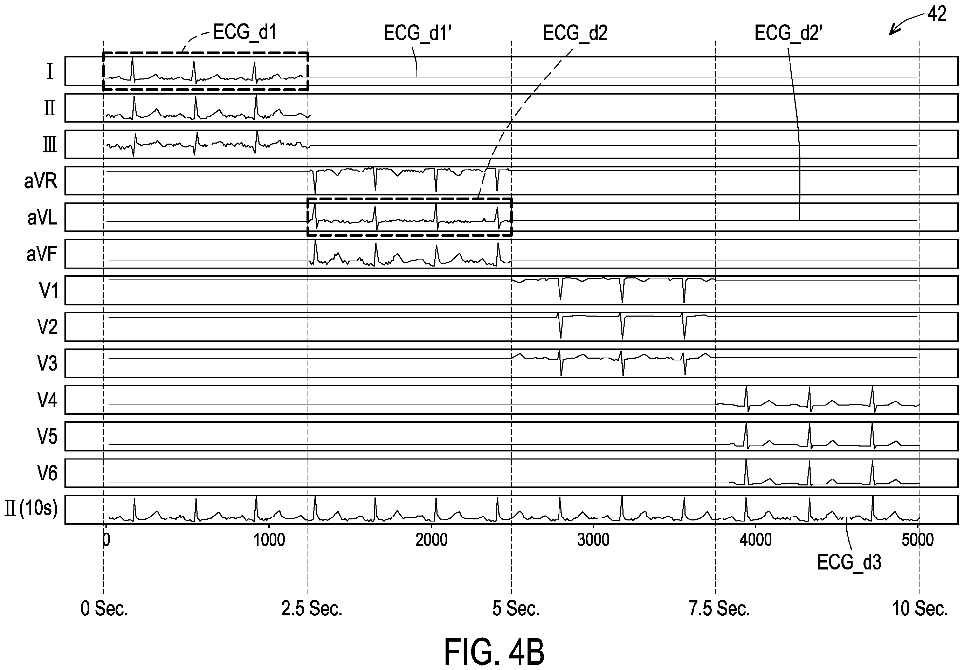

For example, FIG. 4A and FIG. 4B are schematic diagrams of a zero-padding operation and a stacking operation according to an embodiment of the present disclosure. Please refer to FIG. 4A, after performing file format conversion and data retrieval, the processor 120 can obtain 12-lead electrocardiogram data 41. Referring to FIG. 4B, the processor 120 can convert the electrocardiogram data 41 of 12 leads into the integrated ECG data 42.

Please refer to FIG. 4A and FIG. 4B at the same time, after the processor 120 obtains the electrocardiogram data ECG_d1 of the lead I corresponding to the first time span "0 to 2.5 seconds", the processor 120 determines the second time span"2.5 seconds to 10 seconds" other than the first time span "0 seconds to 2.5 seconds" based on the third time span "0 seconds to 10 seconds" of lead II. Therefore, for the electrocardiogram data ECG_d1 of lead I, the processor 120 can perform the zero-padding operation for the second time span "2.5 seconds to 10 seconds". That is, a plurality of zeros can be added to the electrocardiogram data ECG_d1 of the lead I to generate the compensated ECG data ECG_d1' of the lead I. In other words, the compensated ECG data ECG_d1' may include the electrocardiogram data ECG_d1 and a plurality of zeros added at its end. The compensated ECG data ECG_d1' and electrocardiogram data ECG_d3 may be two data arrays with the same data sizes.

Similarly, after processor 120 obtain the electrocardiogram data ECG_d2 of the lead aVL corresponding to the fourth time span "2.5 seconds to 5 seconds", the processor 120 determines the fifth time spans "0 seconds to 2.5 seconds" and "5 seconds to 10 seconds" other than the fourth time span "2.5 seconds to 5 seconds" based on the third time span "0 seconds to 10 seconds" of lead II. Therefore, for the electrocardiogram data ECG_d2 of lead aVL, the processor 120 can perform the zero-padding operation for the fifth time span "0 seconds to 2.5 seconds" and "5 seconds to 10 seconds". That is to add a plurality of zeros to the electrocardiogram data ECG_d2 of the lead aVL to generate the compensated ECG data ECG_d2' of the lead aVL. In other words, the compensated ECG data ECG_d2' may include electrocardiogram data ECG_d2 and the plurality of zeros added at its front and its back. The compensated ECG data ECG_d2' and electrocardiogram data ECG_d3 may be two data arrays with the same data sizes. It should be noted that the second time span "2.5 seconds to 10 seconds" associated with the lead I partially does not overlap with the fifth time span "0 seconds to 2.5 seconds" and "5 seconds to 10 seconds" associated with the lead aVL.

In addition, based on the above description, those skilled in the art can understand how to generate the compensated ECG data of 12 leads according to similar operations. It is not described in details here. Accordingly, after the processor 120 obtains the compensated ECG data respectively corresponding to the 12 leads, the processor 120 can stack the compensated ECG data respectively corresponding to the 12 leads and the electrocardiogram data ECG_d3 of the lead II to generate the integrated ECG data 42. However, the present disclosure does not limit the stacking sequence of the compensated ECG data of these leads, and FIG. 4B is merely an exemplary illustration. It can be seen that, assuming that the compensated ECG data respectively corresponding to the 12 leads is a 1*S data array, the integrated ECG data 42 can be a 13*S data matrix.

In step S240, the processor 120 generates a diagnostic result of the heart state according to the integrated ECG data and the deep learning (DL) model. Specifically, the processor 120 can input the integrated ECG data into the pre-trained deep learning model, so that the deep learning model can output the diagnostic result of the heart state. This deep learning model is, for example, convolutional neural networks (CNN), which includes a convolutional layer and a fully connected layer. In some embodiments, the deep learning model can learn and extract features from the integrated ECG data, and predict the diagnostic result of the heart state accordingly. The diagnostic result of the heart state may include the risk probability of suffering from coronary heart disease or the risk probability of having other heart diseases such as arrhythmia.

In this way, the processor 120 can output the diagnostic result of the heart state for providing to a medical personnel as a reference. In particular, the integrated ECG data in the embodiment of the present disclosure retains the correlation of the electrocardiogram data of the plurality of leads with respect to time, so the accuracy of the deep learning model can be improved. In addition, by converting the electrocardiogram file in the first file format into a SVG file, the processor 120 can obtain the electrocardiogram data closer to the original electrocardiogram measurement results without removing the grid lines of the electrocardiogram paper in the electrocardiogram.

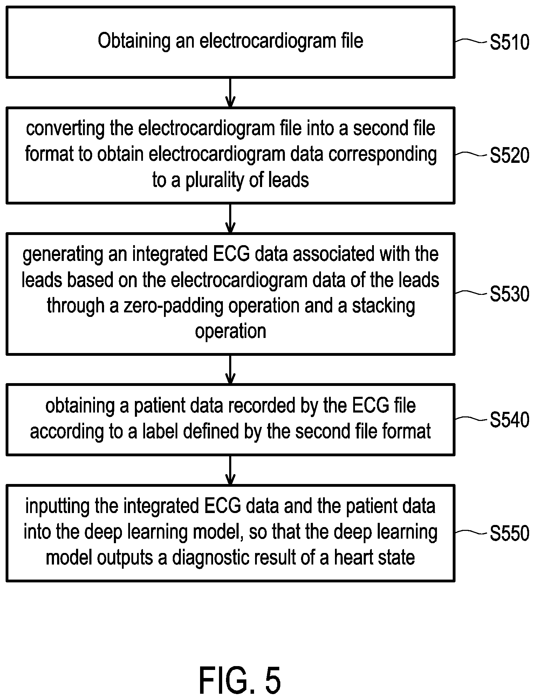

FIG. 5 is a flowchart of a method based on electrocardiogram diagnose heart state according to an embodiment of the present disclosure. Please refer to FIG. 1 and FIG. 5, the method of this embodiment is applicable to the electronic device 100 in the above-mentioned embodiments, and the details of each step in FIG. 5 will be described below in conjunction with various components in the electronic device 100.

In step S510, the processor 120 obtains an electrocardiogram file. In step S520, the processor 120 converts an electrocardiogram file into a second file format and obtains electrocardiogram data corresponding to a plurality of leads. In step S530, the processor 120 generates an integrated ECG data associated with the plurality of leads based on the electrocardiogram data of the plurality of leads through a zero-padding operation and a stacking operation. The detailed operations of step S510 to step S530 are similar to the detailed operations of step S210 to step S230 shown in FIG. 2, and will not be repeated herein.

It should be noted that, in step S540, the processor 120 can obtain the patient data recorded by the electrocardiogram file according to a label defined by the second file format. By analyzing the label in the file in the second file format and the known location information of the patient data in the electrocardiogram file, the processor 120 can obtain the patient data recorded by the electrocardiogram file. For example, assuming that processor 120 converts the electrocardiogram file into a SVG file, the processor 120 can obtain patient data by extracting the text content of the label <tspan> element. For example, patient data, such as patient data P_info shown in FIG. 3, may include age and gender of the patient, and so on.

In step S550, the processor 120 can input the integrated ECG data and the patient data into the deep learning model, so that the deep learning model outputs the diagnostic result of the heart state. That is to say, in some embodiments, the processor 120 can also take the patient information into consideration to determine the risk probability of whether the patient suffers from coronary heart disease. In some embodiments, the processor 120 can also standardize or encode the patient data, so as to convert the patient data into a data format suitable for inputting into the deep learning model. For example, the processor 120 can encode the gender "male" in the patient data as a two-digit code "10", and encode the gender "female" in the patient data as a two-digit code "01". Alternatively, the processor 120 may divide the age in the patient data by 100 to obtain a normalized age value. In one embodiment, the processor 120 may perform a concatenate operation on the patient data processed as above and the integrated ECG data.

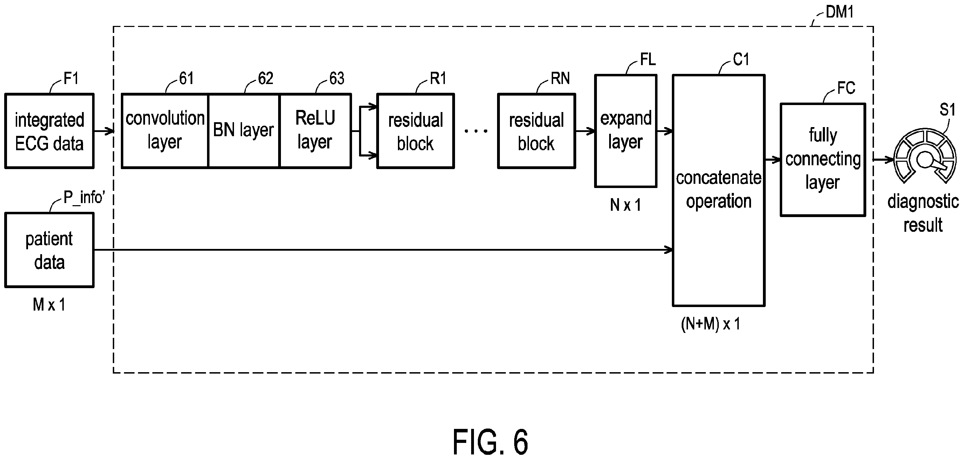

For example, FIG. 6 is a schematic diagram of a deep learning model according to an embodiment of the present disclosure. Referring to FIG. 6, the deep learning model DM1 may include a convolution layer 61, a BN layer 62, a ReLU layer 63, a plurality of residual blocks R1-RN, an expand layer FL, a concatenate operation C1, and a fully connecting layer FC. The convolution layer 61 may use n*n convolution kernels, such as 3*3 convolution kernels, to perform convolution calculation. The BN layer 62 performs batch normalization (BN) operation. The ReLU layer 63 may use a rectified linear unit (ReLU) function as an activation function. The residual blocks R1-RN use a residual learning strategy to combine the output of the front layer with the output of the rear layer. Each residual block R1-RN may include a convolution layer, a pooling layer, and the like.

However, the deep learning model DM1 is only an exemplary description, and the present disclosure does not limit the model architecture of the deep learning model. For example, the number of convolution layers 61 and the number of residual blocks R1-RN can be designed according to actual application situations.

The processor 120 can input the integrated ECG data F1 to the convolution layer 61 so that the residual block RN can output one or more feature vectors. The expand layer FL can spread out the above to generate an N* 1 vector matrix. In addition, the processed patient data P_info' can be represented as an M* 1 vector matrix. The N* 1 vector matrix generated by the expansion layer FL can be concatenated with the patient data P_info' through a concatenate operation C1 to generate a (N+M)* 1 vector matrix. Then, the (N+M)* 1 vector matrix will be input to the fully connecting layer FC, and the fully connecting layer FC can output the diagnostic result S1 of the heart state. For example, the fully connecting layer FC can use the softmax function to perform classification operations.

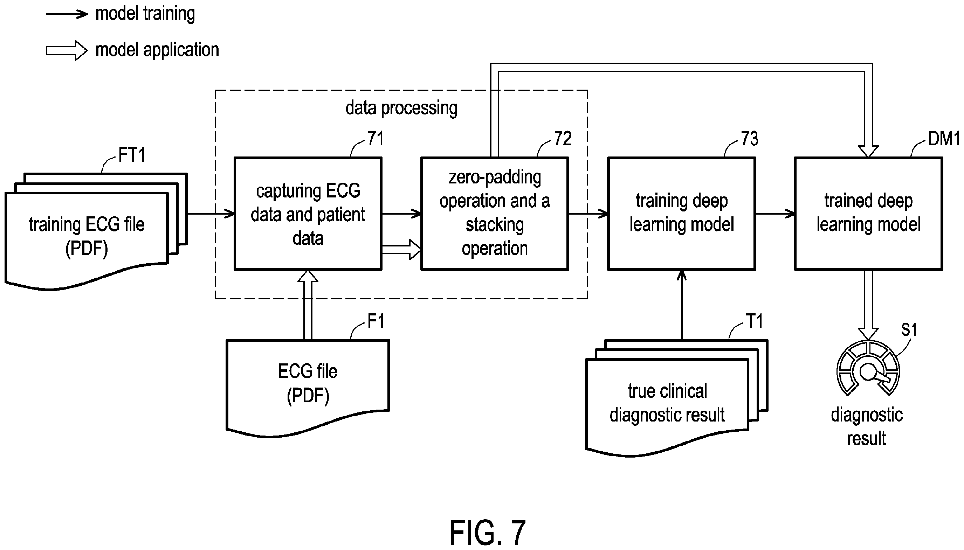

FIG. 7 is a schematic diagram of a training depth model and an application depth model according to an embodiment of the present disclosure. Please refer to FIG. 7, which illustrates the process of training the deep learning model DM1 and applying the deep learning model DM1.

During the training process of the deep learning model DM1, the processor 120 can obtain a plurality of training electrocardiogram files FT1, and these training electrocardiogram files FT1 can all be in PDF file format. Then, in operation 71, the processor 120 can perform file format converting on the training electrocardiogram file FT1 and extracting electrocardiogram data and patient data, and generate an electrocardiogram data of potential traces of a plurality of leads in the training electrocardiogram file FT1. In operation 72, the processor 120 can perform a zero-padding operation and a stacking operation on the electrocardiogram data of the training electrocardiogram file FT1 to obtain an integrated ECG data of the training electrocardiogram file FT1. In operation 73, the processor 120 can use the integrated ECG data and patient data of the training electrocardiogram file FT1 to train the deep learning model DM1. Herein, the processor 120 can train the deep learning model DM1 according to the real clinical diagnostic result T1 corresponding to the training electrocardiogram file FT1. The real clinical diagnostic result T1 is, for example, a marker that medical personnel determines that any coronary artery is blocked by more than 50%, or a marker that determines that there is no coronary artery blockage. In other words, the real clinical diagnostic result T1 may include a first marker of having coronary heart disease or a second marker of a normal state. The model parameters of the trained deep learning model DM1 can be recorded in the storage device 120.

During the application process of the deep learning model DM1, the processor 120 can obtain the electrocardiogram file F1 of the patient to be diagnosed. Next, in operation 71, the processor 120 can perform file format converting on the electrocardiogram file F1 and extracting the electrocardiogram data and patient data, and generate the electrocardiogram data of potential traces of the plurality of leads in the electrocardiogram file F1. In operation 72, the processor 120 may perform the zero-padding operation and the stacking operation on the electrocardiogram data of the electrocardiogram file F1 to obtain the integrated ECG data of the electrocardiogram file F1. The processor 120 can input the integrated ECG data of the electrocardiogram file F1 and the converted data of the patient to be diagnosed to the trained deep learning model DM1, so that the deep learning model DM1 can output the diagnostic result S1, i.e., the risk of suffering from CAD probability, of the heart state.

To sum up, in the embodiment of the present disclosure, the electrocardiogram file can be converted from the first file format to the second file format. By converting the electrocardiogram file in the first file format into a vector graphics file format, the embodiment of the present disclosure can obtain electrocardiogram data that is closer to the original electrocardiogram measurement results without removing grid lines of the electrocardiogram paper in the electrocardiogram. In addition, the integrated ECG data in the embodiment of the present disclosure retains the correlation of electrocardiogram data of a plurality leads with respect to time, and takes patient data into consideration, so the accuracy of the deep learning model in judging the health condition of the coronary arteries of patients can be improved. Thereby, relevant medical personnel can control the condition of the heart more easily, so as to reduce the probability of making wrong assessments.

## Claims

1. A processor implemented method of diagnosing a heart state based on an electrocardiogram, comprising:
obtaining an electrocardiogram file (31, F1), wherein the electrocardiogram file (31, F1) is in a first file format and comprises a plurality of potential traces (ECG_t1,ECG_t2, ECG_t3) of a plurality of leads;
converting the electrocardiogram file (31, F1) into a second file format to obtain electrocardiogram data (41, ECG_d1, ECG_d2, ECG_d3) corresponding to the plurality of leads, wherein, each of the plurality of potential traces (ECG_t1,ECG_t2, ECG_t3) relative to time in the electrocardiogram file (31, F1) is converted into the electrocardiogram data (41, ECG_d1, ECG_d2, ECG_d3) of each of the plurality of lead;
generating an integrated electrocardiogram data (42, F1) associated with the plurality of leads based on the electrocardiogram data (41, ECG_d1, ECG_d2, ECG_d3) of the plurality of leads through a zero-padding operation and a stacking operation; and
generating a diagnostic result (S1) of a heart state according to the integrated electrocardiogram data (42, F1) and a deep learning model (DM1).

2. The method of diagnosing the heart state based on the electrocardiogram as claimed in claim 1, wherein the first file format comprises a portable document format (PDF) file format, and the second file format comprises a scalable sector graphics (SVG) file format.

3. The method of diagnosing the heart state based on the electrocardiogram as claimed in claim 2, wherein the step of converting the electrocardiogram file (31, F1) into the second file format and obtaining the electrocardiogram data (41, ECG_d1, ECG_d2, ECG_d3) corresponding to the plurality of leads comprises:
according to a label defined by the second file format, obtaining the trace description coordinate data corresponding to each of the plurality of leads from a file converted to the second file format, wherein the trace description coordinate data of each of the plurality of leads is used to describe the potential trace (ECG_t1,ECG_t2, ECG_t3) of each of the plurality of leads; and
converting the trace description coordinate data of each of the plurality of leads to the electrocardiogram data (41, ECG_d1, ECG_d2, ECG_d3) corresponding to each of the plurality of leads.

4. The method of diagnosing the heart state based on the electrocardiogram as claimed in claim 1, wherein the step of generating the diagnostic result of the heart state according to the integrated electrocardiogram data (42, F1) and the deep learning model (DM1) comprises:
inputting the integrated electrocardiogram data (42, F1) and patient data (P_info, P_info') into the deep learning model (DM1), so that the deep learning model (DM1) outputs the diagnostic result (S1) of the heart state.

5. The method of diagnosing the heart state based on the electrocardiogram as claimed in claim 4, further comprising:
obtaining the patient data (P_info, P_info') recorded by the electrocardiogram file (31, F1) according to a label defined by the second file format.

6. The method of diagnosing the heart state based on the electrocardiogram as claimed in claim 1, the plurality of leads comprise at least two of lead I, lead II, lead III, lead aVR, lead aVL, lead aVF, lead V1, lead V2, lead V3, lead V4, lead V5, and lead V6.

7. The method of diagnosing the heart state based on the electrocardiogram as claimed in claim 1, the plurality of leads comprise a first lead and a second lead, and the steps of generating the integrated electrocardiogram data (42, F1) associated with the plurality of leads based on the electrocardiogram data (41, ECG_d1, ECG_d2, ECG_d3) of the plurality of leads through the zero-padding operation and the stacking operation comprise:
based on a first time span corresponding to the electrocardiogram data (41, ECG_d1, ECG_d2, ECG_d3) of the first lead, performing the zero-padding operation in at least one second time span other than the first time span to generate a compensated electrocardiogram data (ECG_d1', ECG_d2') of the first lead, wherein the third time span corresponding to the electrocardiogram data of the second lead comprises the first time span and the at least one second time span.

8. The method of diagnosing the heart state based on the electrocardiogram as claimed in claim 7, the plurality of leads comprise a third lead, and the steps of generating the integrated electrocardiogram data (42, F1) associated with the plurality of leads based on the electrocardiogram data (41, ECG_d1, ECG_d2, ECG_d3) of the plurality of leads through the zero-padding operation and the stacking operation comprise:
based on the fourth time span corresponding to the electrocardiogram data (41, ECG_d1, ECG_d2, ECG_d3) of the third lead, performing the zero-padding operation in at least one fifth time span other than the fourth time span to generate the compensated electrocardiogram data (ECG_d1', ECG_d2') of the third lead, wherein the at least one second time span partially not overlap with the at least one fifth time span.

9. The method of diagnosing the heart state based on the electrocardiogram as claimed in claim 7, wherein the step of generating the integrated electrocardiogram data (42, F1) associated with the plurality of leads based on the electrocardiogram data (41, ECG_d1, ECG_d2, ECG_d3) of the plurality of leads through the zero-padding operation and the stacking operation comprises:
generating the integrated electrocardiogram data (42, F1) associated with the plurality of leads by stacking the compensated electrocardiogram data (ECG_d1', ECG_d2') of the first lead and the electrocardiogram data (41, ECG_d1, ECG_d2, ECG_d3) of the second lead.

10. An electronic device, comprising:
a storage device (110); and
a processor (120), coupled to the storage device (110), configured to:
obtain an electrocardiogram file (31, F1), wherein the electrocardiogram file (31, F1) is a first file format;
convert the electrocardiogram file (31, F1) into a second file format to obtain electrocardiogram data (31, F1) corresponding to a plurality of leads, wherein the electrocardiogram data (31, F1) of each of the plurality of leads comprises a potential trace (ECG_t1,ECG_t2, ECG_t3) relative to time;
generate an integrated electrocardiogram data (42, F1) associated with the plurality of leads based on the electrocardiogram data (41, ECG_d1, ECG_d2, ECG_d3) of the plurality of leads through a zero-padding operation and a stacking operation; and
generate a diagnostic result (S1) of a heart state according to the integrated electrocardiogram data (42, F1) and a deep learning model (DM1).

11. The electronic device as claimed in claim 10, wherein the first file format comprises a portable document format (PDF) file format, and the second file format comprises a scalable sector graphics (SVG) file format.

12. The electronic device as claimed in claim 11, wherein the processor (120) is further configured to:
according to a label defined by the second file format, obtain the trace description coordinate data corresponding to each of the plurality of leads from a file converted to the second file format, wherein the trace description coordinate data of each of the plurality of leads is used to describe the potential trace (ECG_t1,ECG_t2, ECG_t3) of each of the plurality of leads; and
convert the trace description coordinate data of each of the plurality of leads to the electrocardiogram data (41, ECG_d1, ECG_d2, ECG_d3) corresponding to each of the plurality of leads.

13. The electronic device as claimed in claim 10, wherein the processor is further configured to:
input the integrated electrocardiogram data (42, F1) and patient data (P_info, P_info') into the deep learning model (DM1), so that the deep learning model (DM1) outputs the diagnostic result (S1) of the heart state.

14. The electronic device as claimed in claim 13, wherein the processor is further configured to:
obtain the patient data (P_info, P_info') recorded by the electrocardiogram file (31, F1) according to a label defined by the second file format.

15. The electronic device as claimed in claim 10, wherein the plurality of leads comprise at least two of lead I, lead II, lead III, lead aVR, lead aVL, lead aVF, lead V1, lead V2, lead V3, lead V4, lead V5, and lead V6.

16. The electronic device as claimed in claim 10, wherein the plurality of leads comprise a first lead and a second lead, and the processor is further configured to:
based on the first time span corresponding to the electrocardiogram data (41, ECG_d1, ECG_d2, ECG_d3) of the first lead, perform the zero-padding operation in at least one second time span other than the first time span to generate a compensated electrocardiogram data (ECG_d1', ECG_d2') of the first lead, wherein the third time span corresponding to the electrocardiogram data (41, ECG_d1, ECG_d2, ECG_d3) of the second lead comprises the first time span and the at least one second time span.

17. The electronic device as claimed in claim 16, wherein the plurality of leads comprise a third lead, and the processor (120) is further configured to:
based on the fourth time span corresponding to the electrocardiogram data (41, ECG_d1, ECG_d2, ECG_d3) of the third lead, perform the zero-padding operation in at least one fifth time span other than the fourth time span to generate the compensated electrocardiogram data (ECG_d1', ECG_d2') of the third lead, wherein the at least one second time span partially not overlap with the at least one fifth time span.

18. The electronic device as claimed in claim 16, wherein the processor is further configured to:
generate the integrated electrocardiogram data (42, F1) associated with the plurality of leads by stacking the compensated electrocardiogram data (ECG_d1', ECG_d2') of the first lead and the electrocardiogram data (41, ECG_d1, ECG_d2, ECG_d3) of the second lead.

## Patentansprüche

1. Prozessorimplementiertes Verfahren zur Diagnose eines Herzzustands auf Basis eines Elektrokardiogramms, umfassend:
Erhalten einer Elektrokardiogrammdatei (31, F1), wobei die Elektrokardiogrammdatei (31, F1) in einem ersten Dateiformat vorliegt und eine Vielzahl von Potentialkurven (ECG_t1, ECG_t2, ECG_t3) einer Vielzahl von Ableitungen umfasst;
Umwandeln der Elektrokardiogrammdatei (31, F1) in ein zweites Dateiformat, um Elektrokardiogrammdaten (41, ECG_d1, ECG_d2, ECG_d3) zu erhalten, die der Vielzahl von Ableitungen entsprechen, wobei jede der Vielzahl von Potentialkurven (ECG_t1, ECG_t2, ECG_t3) relativ zu einer Zeit in der Elektrokardiogrammdatei (31, F1) in die Elektrokardiogrammdaten (41, ECG_d1, ECG_d2, ECG_d3) jeder der Vielzahl von Ableitungen umgewandelt wird;
Erzeugen von der Vielzahl von Ableitungen zugeordneten integrierten Elektrokardiogrammdaten (42, F1) auf Basis der Elektrokardiogrammdaten (41, ECG_d1, ECG_d2, ECG_d3) der Vielzahl von Ableitungen durch einen Zero-Padding-Vorgang und einen Stapelvorgang; und
Erzeugen eines Diagnoseergebnisses (S1) eines Herzzustands gemäß den integrierten Elektrokardiogrammdaten (42, F1) und einem Deep-Learning-Modell (DM1).

2. Verfahren zur Diagnose des Herzzustands auf Basis des Elektrokardiogramms nach Anspruch 1, wobei das erste Dateiformat ein Dateiformat portables Dokumentenformat (PDF) umfasst und das zweite Dateiformat ein Dateiformat skalierbare Sektorgrafik (SVG) umfasst.

3. Verfahren zur Diagnose des Herzzustands auf Basis des Elektrokardiogramms nach Anspruch 2, wobei der Schritt des Umwandelns der Elektrokardiogrammdatei (31, F1) in das zweite Dateiformat und des Erhaltens der Elektrokardiogrammdaten (41, ECG_d1, ECG_d2, ECG_d3), die der Vielzahl von Ableitungen entsprechen, Folgendes umfasst:
Erhalten der Kurvenbeschreibungs-Koordinatendaten, die jeder der Vielzahl von Ableitungen entsprechen, aus einer in das zweite Dateiformat umgewandelten Datei gemäß einem durch das zweite Dateiformat definierten Label, wobei die Kurvenbeschreibungs-Koordinatendaten jeder der Vielzahl von Ableitungen zum Beschreiben der Potentialkurven (ECG_t1, ECG_t2, ECG_t3) jeder der Vielzahl von Ableitungen verwendet werden; und
Umwandeln der Kurvenbeschreibungs-Koordinatendaten jeder der Vielzahl von Ableitungen in die Elektrokardiogrammdaten (41, ECG_d1, ECG_d2, ECG_d3), die jeder der Vielzahl von Ableitungen entsprechen.

4. Verfahren zur Diagnose des Herzzustands auf Basis des Elektrokardiogramms nach Anspruch 1, wobei der Schritt des Erzeugens des Diagnoseergebnisses des Herzzustands gemäß den integrierten Elektrokardiogrammdaten (42, F1) und dem Deep-Learning-Modell (DM1) Folgendes umfasst:
Eingeben der integrierten Elektrokardiogrammdaten (42, F1) und von Patientendaten (P_info, P_info') in das Deep-Learning-Modell (DM1), sodass das Deep-Learning-Modell (DM1) das Diagnoseergebnis (S1) des Herzzustands ausgibt.

5. Verfahren zur Diagnose des Herzzustands auf Basis des Elektrokardiogramms nach Anspruch 4, ferner umfassend:
Erhalten der von der Elektrokardiogrammdatei (31, F1) aufgezeichneten Patientendaten (P_info, P_info') gemäß einem durch das zweite Dateiformat definierten Label.

6. Verfahren zur Diagnose des Herzzustands auf Basis des Elektrokardiogramms nach Anspruch 1, wobei die Vielzahl von Ableitungen mindestens zwei von Ableitung I, Ableitung II, Ableitung III, Ableitung aVR, Ableitung aVL, Ableitung aVF, Ableitung V1, Ableitung V2, Ableitung V3, Ableitung V4, Ableitung V5 und Ableitung V6 umfasst.

7. Verfahren zur Diagnose des Herzzustands auf Basis des Elektrokardiogramms nach Anspruch 1, wobei die Vielzahl von Ableitungen eine erste Ableitung und eine zweite Ableitung umfasst und die Schritte des Erzeugens der der Vielzahl von Ableitungen zugeordneten integrierten Elektrokardiogrammdaten (42, F1) auf Basis der Elektrokardiogrammdaten (41, ECG_d1, ECG_d2, ECG_d3) der Vielzahl von Ableitungen durch den Zero-Padding-Vorgang und den Stapelvorgang Folgendes umfassen:
Durchführen des Zero-Padding-Vorgangs auf Basis einer ersten Zeitspanne, die den Elektrokardiogrammdaten (41, ECG_d1, ECG_d2, ECG_d3) der ersten Ableitung entspricht, über mindestens eine zweite Zeitspanne, die sich von der ersten Zeitspanne unterscheidet, um kompensierte Elektrokardiogrammdaten (ECG_d1', ECG_d2') der ersten Ableitung zu erzeugen, wobei die dritte Zeitspanne, die den Elektrokardiogrammdaten der zweiten Ableitung entspricht die erste Zeitspanne und die mindestens eine zweite Zeitspanne umfasst.

8. Verfahren zur Diagnose des Herzzustands auf Basis des Elektrokardiogramms nach Anspruch 7, wobei die Vielzahl von Ableitungen eine dritte Ableitung umfasst und die Schritte des Erzeugens der der Vielzahl von Ableitungen zugeordneten integrierten Elektrokardiogrammdaten (42, F1) auf Basis der Elektrokardiogrammdaten (41, ECG_d1, ECG_d2, ECG_d3) der Vielzahl von Ableitungen durch den Zero-Padding-Vorgang und den Stapelvorgang Folgendes umfassen:
Durchführen des Zero-Padding-Vorgangs auf Basis der vierten Zeitspanne, die den Elektrokardiogrammdaten (41, ECG_d1, ECG_d2, ECG_d3) der dritten Ableitung entspricht, über mindestens eine fünfte Zeitspanne, die sich von der vierten Zeitspanne unterscheidet, um die kompensierten Elektrokardiogrammdaten (ECG_d1', ECG_d2') der dritten Ableitung zu erzeugen, wobei die mindestens eine zweite Zeitspanne teilweise nicht die mindestens eine fünfte Zeitspanne überlappt.

9. Verfahren zur Diagnose des Herzzustands auf Basis des Elektrokardiogramms nach Anspruch 7, wobei der Schritt des Erzeugens der der Vielzahl von Ableitungen zugeordneten integrierten Elektrokardiogrammdaten (42, F1) auf Basis der Elektrokardiogrammdaten (41, ECG_d1, ECG_d2, ECG_d3) der Vielzahl von Ableitungen durch den Zero-Padding-Vorgang und den Stapelvorgang Folgendes umfasst:
Erzeugen der der Vielzahl von Ableitungen zugeordneten integrierten Elektrokardiogrammdaten (42, F1) durch Stapeln der kompensierten Elektrokardiogrammdaten (ECG_d1', ECG_d2') der ersten Ableitung und der Elektrokardiogrammdaten (41, ECG_d1, ECG_d2, ECG_d3) der zweiten Ableitung.

10. Elektronische Vorrichtung, umfassend:
eine Speichervorrichtung (110); und
einen Prozessor (120), der an die Speichervorrichtung (110) gekoppelt ist, konfiguriert zum:
Erhalten einer Elektrokardiogrammdatei (31, F1), wobei die Elektrokardiogrammdatei (31, F1) in einem ersten Dateiformat vorliegt;
Umwandeln der Elektrokardiogrammdatei (31, F1) in ein zweites Dateiformat, um Elektrokardiogrammdaten (31, F1) zu erhalten, die einer Vielzahl von Ableitungen entsprechen, wobei die Elektrokardiogrammdaten (31, F1) jeder der Vielzahl von Ableitungen eine Potentialkurve (ECG_t1, ECG_t2, ECG_t3) relativ zu Zeit umfassen;
Erzeugen von der Vielzahl von Ableitungen zugeordneten integrierten Elektrokardiogrammdaten (42, F1) auf Basis der Elektrokardiogrammdaten (41, ECG_d1, ECG_d2, ECG_d3) der Vielzahl von Ableitungen durch einen Zero-Padding-Vorgang und einen Stapelvorgang; und
Erzeugen eines Diagnoseergebnisses (S1) eines Herzzustands gemäß den integrierten Elektrokardiogrammdaten (42, F1) und einem Deep-Learning-Modell (DM1).

11. Elektronische Vorrichtung nach Anspruch 10, wobei das erste Dateiformat ein Dateiformat portables Dokumentenformat (PDF) umfasst und das zweite Dateiformat ein Dateiformat skalierbare Sektorgrafik (SVG) umfasst.

12. Elektronische Vorrichtung nach Anspruch 11, wobei der Prozessor (120) zu Folgendem konfiguriert ist:
Erhalten der Kurvenbeschreibungs-Koordinatendaten, die jeder der Vielzahl von Ableitungen entsprechen, aus einer in das zweite Dateiformat umgewandelten Datei gemäß einem durch das zweite Dateiformat definierten Label, wobei die Kurvenbeschreibungs-Koordinatendaten jeder der Vielzahl von Ableitungen zum Beschreiben der Potentialkurven (ECG_t1, ECG_t2, ECG_t3) jeder der Vielzahl von Ableitungen verwendet werden; und
Umwandeln der Kurvenbeschreibungs-Koordinatendaten jeder der Vielzahl von Ableitungen in die Elektrokardiogrammdaten (41, ECG_d1, ECG_d2, ECG_d3), die jeder der Vielzahl von Ableitungen entsprechen.

13. Elektronische Vorrichtung nach Anspruch 10, wobei der Prozessor ferner zu Folgendem konfiguriert ist:
Eingeben der integrierten Elektrokardiogrammdaten (42, F1) und von Patientendaten (P_info, P_info') in das Deep-Learning-Modell (DM1), sodass das Deep-Learning-Modell (DM1) das Diagnoseergebnis (S1) des Herzzustands ausgibt.

14. Elektronische Vorrichtung nach Anspruch 13, wobei der Prozessor ferner zu Folgendem konfiguriert ist:
Erhalten der von der Elektrokardiogrammdatei (31, F1) aufgezeichneten Patientendaten (P_info, P_info') gemäß einem durch das zweite Dateiformat definierten Label.

15. Elektronische Vorrichtung nach Anspruch 10, wobei die Vielzahl von Ableitungen mindestens zwei von Ableitung I, Ableitung II, Ableitung III, Ableitung aVR, Ableitung aVL, Ableitung aVF, Ableitung V1, Ableitung V2, Ableitung V3, Ableitung V4, Ableitung V5 und Ableitung V6 umfasst.

16. Elektronische Vorrichtung nach Anspruch 10, wobei die Vielzahl von Ableitungen eine erste Ableitung und eine zweite Ableitung umfasst und der Prozessor ferner konfiguriert ist zum:
Durchführen des Zero-Padding-Vorgangs auf Basis einer ersten Zeitspanne, die den Elektrokardiogrammdaten (41, ECG_d1, ECG_d2, ECG_d3) der ersten Ableitung entspricht, über mindestens eine zweite Zeitspanne, die sich von der ersten Zeitspanne unterscheidet, um kompensierte Elektrokardiogrammdaten (ECG_d1', ECG_d2') der ersten Ableitung zu erzeugen, wobei die dritte Zeitspanne, die den Elektrokardiogrammdaten (41, ECG_d1, ECG_d2, ECG_d3) der zweiten Ableitung entspricht die erste Zeitspanne und die mindestens eine zweite Zeitspanne umfasst.

17. Elektronische Vorrichtung nach Anspruch 16, wobei die Vielzahl von Ableitungen eine dritte Ableitung umfasst und der Prozessor (120) ferner konfiguriert ist zum:
Durchführen des Zero-Padding-Vorgangs auf Basis der vierten Zeitspanne, die den Elektrokardiogrammdaten (41, ECG_d1, ECG_d2, ECG_d3) der dritten Ableitung entspricht, über mindestens eine fünfte Zeitspanne, die sich von der vierten Zeitspanne unterscheidet, um die kompensierten Elektrokardiogrammdaten (ECG_d1', ECG_d2') der dritten Ableitung zu erzeugen, wobei die mindestens eine zweite Zeitspanne teilweise nicht die mindestens eine fünfte Zeitspanne überlappt.

18. Elektronische Vorrichtung nach Anspruch 16, wobei der Prozessor ferner zu Folgendem konfiguriert ist:
Erzeugen der der Vielzahl von Ableitungen zugeordneten integrierten Elektrokardiogrammdaten (42, F1) durch Stapeln der kompensierten Elektrokardiogrammdaten (ECG_d1', ECG_d2') der ersten Ableitung und der Elektrokardiogrammdaten (41, ECG_d1, ECG_d2, ECG_d3) der zweiten Ableitung.

## Revendications

1. Procédé mis en œuvre par un processeur pour diagnostiquer un état cardiaque sur la base d'un électrocardiogramme, comprenant :
l'obtention d'un fichier d'électrocardiogramme (31, F1), dans lequel le fichier d'électrocardiogramme (31, F1) est dans un premier format de fichier et comprend une pluralité de traces potentielles (ECG_t1, ECG_t2, ECG_t3) d'une pluralité de dérivations ;
la conversion du fichier d'électrocardiogramme (31, F1) en un second format de fichier pour obtenir des données d'électrocardiogramme (41, ECG_d1, ECG_d2, ECG_d3) correspondant à la pluralité de dérivations, dans lequel chacune de la pluralité de traces potentielles (ECG_t1, ECG_t2, ECG_t3) par rapport au temps dans le fichier d'électrocardiogramme (31, F1) est convertie en données d'électrocardiogramme (41, ECG_d1, ECG_d2, ECG_d3) de chacune de la pluralité de dérivations ;
la génération des données d'électrocardiogramme intégrées (42, F1) associées à la pluralité de dérivations sur la base des données d'électrocardiogramme (41, ECG_d1, ECG_d2, ECG_d3) de la pluralité de dérivations par une opération de remplissage de zéros et une opération d'empilement ; et
la génération d'un résultat de diagnostic (S1) d'un état cardiaque selon les données d'électrocardiogramme intégrées (42, F1) et d'un modèle d'apprentissage profond (DM1).

2. Procédé de diagnostic d'état cardiaque sur la base d'électrocardiogramme selon la revendication 1, dans lequel le premier format de fichier comprend un format de fichier au format de document portable (PDF), et le second format de fichier comprend un format de fichier graphique vectoriel adaptable (SVG).

3. Procédé de diagnostic d'état cardiaque sur la base d'électrocardiogramme selon la revendication 2, dans lequel l'étape de conversion du fichier d'électrocardiogramme (31, F1) dans le second format de fichier et d'obtention des données d'électrocardiogramme (41, ECG_d1, ECG_d2, ECG_d3) correspondant à la pluralité de dérivations comprend :
selon une étiquette définie par le second format de fichier, l'obtention des données de coordonnées de description de trace correspondant à chacune de la pluralité de dérivations à partir d'un fichier converti au second format de fichier, dans lequel les données de coordonnées de description de trace de chacune de la pluralité de dérivations sont utilisées pour décrire la trace potentielle (ECG_t1, ECG_t2, ECG_t3) de chacune de la pluralité de dérivations ; et
la conversion des données de coordonnées de description de trace de chacune de la pluralité de dérivations en données d'électrocardiogramme (41, ECG_d1, ECG_d2, ECG_d3) correspondant à chacune de la pluralité de dérivations.

4. Procédé de diagnostic d'état cardiaque sur la base d'électrocardiogramme selon la revendication 1, dans lequel l'étape de génération du résultat de diagnostic de l'état cardiaque selon les données d'électrocardiogramme intégrées (42, F1) et le modèle d'apprentissage profond (DM1) comprend :
l'entrée des données d'électrocardiogramme intégrées (42, F1) et des données du patient (P_info, P_info') dans le modèle d'apprentissage profond (DM1), de sorte que le modèle d'apprentissage profond (DM1) génère le résultat de diagnostic (S1) de l'état cardiaque.

5. Procédé de diagnostic d'état cardiaque sur la base d'électrocardiogramme selon la revendication 4, comprenant également :
l'obtention des données du patient (P_info, P_info') enregistrées par le fichier d'électrocardiogramme (31, F1) selon une étiquette définie par le second format de fichier.

6. Procédé de diagnostic d'état cardiaque sur la base d'électrocardiogramme selon la revendication 1, la pluralité de dérivations comprenant au moins deux parmi la dérivation I, la dérivation II, la dérivation III, la dérivation aVR, la dérivation aVL, la dérivation aVF, la dérivation V1, la dérivation V2, la dérivation V3, la dérivation V4, la dérivation V5 et la dérivation V6.

7. Procédé de diagnostic d'état cardiaque sur la base d'électrocardiogramme selon la revendication 1, la pluralité de dérivations comprenant une première dérivation et une deuxième dérivation, et les étapes de génération des données d'électrocardiogramme intégrées (42, F1) associées à la pluralité de dérivations basées sur les données d'électrocardiogramme (41, ECG_d1, ECG_d2, ECG_d3) de la pluralité de dérivations par l'opération de remplissage de zéros et l'opération d'empilement comprenant :
sur la base d'un premier intervalle de temps correspondant aux données d'électrocardiogramme (41, ECG_d1, ECG_d2, ECG_d3) de la première dérivation, la réalisation de l'opération de remplissage de zéros dans au moins un deuxième intervalle de temps autre que le premier intervalle de temps pour générer des données d'électrocardiogramme compensées (ECG_d1', ECG_d2') de la première dérivation, dans lequel le troisième intervalle de temps correspondant aux données d'électrocardiogramme de la deuxième dérivation comprend le premier intervalle de temps et l'au moins un deuxième intervalle de temps.

8. Procédé de diagnostic d'état cardiaque sur la base d'électrocardiogramme selon la revendication 7, la pluralité de dérivations comprenant une troisième dérivation, et les étapes de génération des données d'électrocardiogramme intégrées (42, F1) associées à la pluralité de dérivations basées sur les données d'électrocardiogramme (41, ECG_d1, ECG_d2, ECG_d3) de la pluralité de dérivations par l'opération de remplissage de zéros et l'opération d'empilement comprenant :
sur la base du quatrième intervalle de temps correspondant aux données d'électrocardiogramme (41, ECG_d1, ECG_d2, ECG_d3) de la troisième dérivation, la réalisation de l'opération de remplissage de zéros dans au moins un cinquième intervalle de temps autre que le quatrième intervalle de temps pour générer les données d'électrocardiogramme compensées (ECG_d1', ECG_d2') de la troisième dérivation, dans lequel l'au moins un deuxième intervalle de temps ne chevauche pas partiellement l'au moins un cinquième intervalle de temps.

9. Procédé de diagnostic d'état cardiaque sur la base d'électrocardiogramme selon la revendication 7, dans lequel l'étape de génération des données d'électrocardiogramme intégrées (42, F1) associées à la pluralité de dérivations basées sur les données d'électrocardiogramme (41, ECG_d1, ECG_d2, ECG_d3) de la pluralité de dérivations par l'opération de remplissage de zéros et l'opération d'empilement comprend :
la génération des données d'électrocardiogramme intégrées (42, F1) associées à la pluralité de dérivations en empilant les données d'électrocardiogramme compensées (ECG_d1', ECG_d2') de la première dérivation et les données d'électrocardiogramme (41, ECG_d1, ECG_d2, ECG_d3) de la deuxième dérivation.

10. Dispositif électronique comprenant :
un dispositif de stockage (110) ; et
un processeur (120), couplé au dispositif de stockage (110),
configuré pour :
obtenir un fichier d'électrocardiogramme (31, F1), dans lequel le fichier d'électrocardiogramme (31, F1) est un premier format de fichier ;
convertir le fichier d'électrocardiogramme (31, F1) en un second format de fichier pour obtenir des données d'électrocardiogramme (31, F1) correspondant à une pluralité de dérivations, dans lequel les données d'électrocardiogramme (31, F1) de chacune de la pluralité de dérivations comprend une trace potentielle (ECG_t1, ECG_t2, ECG_t3) par rapport au temps ;
générer des données d'électrocardiogramme intégrées (42, F1) associées à la pluralité de dérivations sur la base des données d'électrocardiogramme (41, ECG_d1, ECG_d2, ECG_d3) de la pluralité de dérivations par une opération de remplissage de zéros et une opération d'empilement ; et
générer un résultat de diagnostic (S1) d'un état cardiaque selon les données d'électrocardiogramme intégrées (42, F1) et un modèle d'apprentissage profond (DM1).

11. Dispositif électronique selon la revendication 10, dans lequel le premier format de fichier comprend un format de fichier au format de document portable (PDF), et le second format de fichier comprend un format de fichier graphique vectoriel adaptable (SVG).

12. Dispositif électronique selon la revendication 11, dans lequel le processeur (120) est également configuré pour :
selon une étiquette définie par le second format de fichier, obtenir les données de coordonnées de description de trace correspondant à chacune de la pluralité de dérivations à partir d'un fichier converti au second format de fichier, dans lequel les données de coordonnées de description de trace de chacune de la pluralité de dérivations sont utilisées pour décrire la trace potentielle (ECG_t1, ECG_t2, ECG_t3) de chacune de la pluralité de dérivations ; et
convertir les données de coordonnées de description de trace de chacune de la pluralité de dérivations en données d'électrocardiogramme (41, ECG_d1, ECG_d2, ECG_d3) correspondant à chacune de la pluralité de dérivations.

13. Dispositif électronique selon la revendication 10, dans lequel le processeur est également configuré pour :
entrer les données d'électrocardiogramme intégrées (42, F1) et les données du patient (P_info, P_info') dans le modèle d'apprentissage profond (DM1), de sorte que le modèle d'apprentissage profond (DM1) génère le résultat de diagnostic (S1) de l'état cardiaque.

14. Dispositif électronique selon la revendication 13, dans lequel le processeur est également configuré pour :
obtenir les données du patient (P_info, P_info') enregistrées par le fichier d'électrocardiogramme (31, F1) selon une étiquette définie par le second format de fichier.

15. Dispositif électronique selon la revendication 10, dans lequel la pluralité de dérivations comprennent au moins deux parmi la dérivation I, la dérivation II, la dérivation III, la dérivation aVR, la dérivation aVL, la dérivation aVF, la dérivation V1, la dérivation V2, la dérivation V3, la dérivation V4, la dérivation V5 et la dérivation V6.

16. Dispositif électronique selon la revendication 10, dans lequel la pluralité de dérivations comprennent une première dérivation et une deuxième dérivation, et le processeur est également configuré pour :
sur la base d'un premier intervalle de temps correspondant aux données d'électrocardiogramme (41, ECG_d1, ECG_d2, ECG_d3) de la première dérivation, réaliser l'opération de remplissage de zéros dans au moins un deuxième intervalle de temps autre que le premier intervalle de temps pour générer des données d'électrocardiogramme compensées (ECG_d1', ECG_d2') de la première dérivation, dans lequel le troisième intervalle de temps correspondant aux données d'électrocardiogramme (41, ECG_d1, ECG_d2, ECG_d3) de la deuxième dérivation comprend le premier intervalle de temps et l'au moins un deuxième intervalle de temps.

17. Dispositif électronique selon la revendication 16, dans lequel la pluralité de dérivations comprennent une troisième dérivation, et le processeur (120) est également configuré pour :
sur la base du quatrième intervalle de temps correspondant aux données d'électrocardiogramme (41, ECG_d1, ECG_d2, ECG_d3) de la troisième dérivation, réaliser l'opération de remplissage de zéros dans au moins un cinquième intervalle de temps autre que le quatrième intervalle de temps pour générer les données d'électrocardiogramme compensées (ECG_d1', ECG_d2') de la troisième dérivation, dans lequel l'au moins un deuxième intervalle de temps ne chevauche pas partiellement l'au moins un cinquième intervalle de temps.

18. Dispositif électronique selon la revendication 16, dans lequel le processeur est également configuré pour :
générer les données d'électrocardiogramme intégrées (42, F1) associées à la pluralité de dérivations en empilant les données d'électrocardiogramme compensées (ECG_d1', ECG_d2') de la première dérivation et les données d'électrocardiogramme (41, ECG_d1, ECG_d2, ECG_d3) de la deuxième dérivation.
